# EUROPEAN PATENT APPLICATION

(11) **EP 4 364 662 A1**
(43) Date of publication of application: **08.05.2024**
(21) Application number: 22205556.8
(22) Date of filing: 04.11.2022
(51) Int. Cl.: A61B 5/16

(54) **COGNITIVE ASSESSMENT OF A SUBJECT**

(71) Applicant: Koninklijke Philips N.V., 5656 AG Eindhoven (NL)
(72) Inventor: VAN DOOREN, Marieke, Eindhoven (NL); VAN EE, Raymond, Eindhoven (NL); SPELT, Hanne Adriana Alijda, 5656AG Eindhoven (NL); DENISSEN, Adrianus Johannes Maria, 5656AG Eindhoven (NL); KLAMING, Laura, Eindhoven (NL); LEUFKENS, Timmy Robertus Maria, Eindhoven (NL)
(74) Representative: Philips Intellectual Property & Standards

(57) **Abstract**

Proposed are concepts pertaining to aiding and/or improving cognitive assessment of a subject. In particular, embodiments propose determining an alertness value describing an alertness level of the subject during the cognitive test. Determination of such an alertness value may provide valuable information for interpreting or understanding the subject's performance in executing a cognitive task. Understanding an alertness level of the subject may also facilitate adaptation of the cognitive assessment, so as to optimize accuracy of the cognitive assessment.

## Description

### FIELD OF THE INVENTION

The present invention relates to the field of cognitive assessment, and in particular to the field of assessing cognitive function or performance of a subject (i.e. individual or patient).

### BACKGROUND OF THE INVENTION

In the field of cognitive assessment, specialized tests are used to assess the cognitive health of subjects. For example, a subject is instructed to undertake a task that is carefully designed to exercise certain cognitive functions. The subject's performance on the task provides insights that a professional (e.g., a medical professional) can use to assess the subject's cognitive health. Examples of cognitive capabilities that are commonly assessed are memory, learning, inductive reasoning, and decision making. Examples of cognitive tests include the clock drawing test, the Montreal Cognitive Assessment (MoCA), the Mini-Mental State Exam (MMSE), and the Mini-Cog.

Distractions during cognitive assessments will cause a drop in the subject's performance, which can lead to inaccurate assessment results. However, given the increasing need for remote healthcare and remote cognitive assessment/testing, it may not always be possible to conduct a cognitive assessment in a controlled environment. Thus, there is a need to optimize the circumstances for a subject to take a cognitive assessment and/or to improve accuracy of a cognitive assessment in view of factors that may affect a subject's performance.

### SUMMARY OF THE INVENTION

The invention is defined by the claims.

According to examples in accordance with an aspect of the invention, there is provided a method for cognitive assessment of a subject, the method comprising:
providing a cognitive test to the subject, the cognitive test prompting execution of a cognitive task for cognitive assessment;
obtaining task execution data describing at least one of: a measure of the subject's performance in executing the cognitive task; and a variation of a physiological parameter of the subject during execution of the cognitive task; and
analyzing the task execution data to determine an alertness value describing an alertness level of the subject during the cognitive test.

Proposed concepts thus aim to provide schemes, solutions, concepts, designs, methods and systems pertaining to aiding and/or improving cognitive assessment of a subject. In particular, embodiments of the invention propose determining an alertness value describing an alertness level of the subject during the cognitive test. Determination of such an alertness value may provide valuable information for interpreting or understanding the subject's performance in executing a cognitive task. Understanding an alertness level of the subject may also facilitate adaptation of the cognitive assessment, so as to optimize accuracy of the cognitive assessment. For instance, a determined alertness value may be used to adapt or modify a workflow of neuropsychological test administration, potentially in real time.

It is proposed that distractions coincide with a drop in alertness level. In addition, neurological conditions such as dementia or stroke may potentially affect alertness level. Embodiments therefore propose to concepts for obtaining an insight into the alertness of a subject during performance of a cognitive test. Such insight(s) about the subject's alertness level may then be used to optimize the circumstances for a subject to perform a cognitive test or assessment. Information about the subject's alertness level may be leveraged to compensate or correct for one or more drops in performance during a cognitive assessment. In this way, proposed embodiments may facilitate improved (i.e. more accurate) cognitive assessment of a subj ect.

Purely by way of example, proposed embodiments may determine a subject's level of alertness during their performance of a cognitive test, and this may then be provided as an additional output and/or used to modify a subsequent cognitive assessment. When provides as an additional output (e.g. as an information signal describing an alertness level of the subject during the cognitive test), it may be employed as a tool for analyzing or interpreting cognitive assessment results for the subject. For instance, information describing an alertness level of the subject may be used to compensate or correct cognitive assessment results. In this way, embodiments may provide a useful for improving analysis and interpretation of neuropsychological testing.

In other words, embodiments propose to generate information describing an alertness level of the subject during the cognitive test. The generated information may aid clinical decision making. For example, embodiments may be used in relation to treatment selection so as support a medical professional when selecting treatment for a subject. Such embodiments may also support clinical planning. Improved Clinical Decision Support (CDS) may therefore be provided by proposed concepts.

Some embodiments may further comprise generating, based on the determined alertness value, an alert for indicating an alertness level of the subject. In this way, a user or system may be alerted when an alertness level of the subject meets a predetermined requirement (e.g. fall below a minimum required level). This may, for example, facilitate timely intervention to ensure that a cognitive assessment if performed in an improved or optimal manner.

An embodiment may comprise, determining, based on the determined alertness value, a modification or correction to the subject's execution of at the cognitive task. Such an embodiment may thus facilitate dynamic and/or automatic adaptation of the executed task in order to facilitate the provision of more accurate results.

Some embodiments may further comprise determining, based on the determined alertness value, a cognitive testing recommendation describing provision of a further cognitive test for the subject. For example, the cognitive testing recommendation may comprise information describing at least one of: an identifier of the further cognitive test; a timing of provision of the further cognitive test; a modification to a cognitive test to create the further cognitive test a physical task. In this way, dynamic and/or automatic adaptation of a cognitive assessment workflow may be enabled, so as to ensure that cognitive assessment is performed in an accurate manner for example.

An exemplary embodiment may comprise determining, based on the determined alertness value, a reliability value describing the reliability of the subject's execution of the cognitive task. This may assist understanding and analysis of assessment results, thereby improving accuracy of cognitive assessment.

In an embodiment, the physiological parameter of the subject may comprise: eye movement; gaze direction; blinking rate; pupil size; eyelid opening and closing speed; vertical eyelid separation distance; heart rate; blood pressure; respiratory interval; posture; brain activity. Data from various known physiological parameter sensors may thus be leveraged by embodiments.

The task execution data may describe a measure of the subject's performance in executing the cognitive task. Analyzing the task execution data may then comprise: comparing the measure of the subject's performance with a reference performance value to determine an alertness value. By way of example, the reference performance value may be based on a historic measure of the subject's performance in executing a previous cognitive task.

That is, proposed embodiments may propose to determine a subject's alertness level by analyzing the subject's performance of a cognitive test and/or by employing tracking technology, like for example known pupillary measures (wherein it is proposed that pupil diameter correlates with alertness).

In some embodiments, an alertness value may be selected from a group comprising: fatigued/drowsy, bored, engaged, distracted, overloaded/stressed, and impaired. In this way, the alertness value may be classified into one of a set of classifications for ease of understanding and/or assessment.

In an embodiment, the steps of obtaining and analyzing task execution data may be undertaken in real time. In this way, real-time information regarding the alertness level of the subject may be provided, thereby enabling quick/prompt assessment and/or action.

According to another aspect, there is provided a computer program product for cognitive assessment of a subject, wherein the computer program product comprises a computer-readable storage medium having computer-readable program code embodied therewith, the computer-readable program code configured to perform all of the steps of a proposed embodiment.

Thus, there may also be provided a computer system comprising: a computer program product according to proposed embodiment; and one or more processors adapted to perform a method according to a proposed concept by execution of the computer-readable program code of said computer program product.

According to still another aspect of the invention, there is provided a system for cognitive assessment of a subject, the system comprising:
an input interface configured to obtain task execution data describing at least one of: a measure of the subject's performance in executing a cognitive task for cognitive assessment prompted by a cognitive test; and a variation of a physiological parameter of the subject during execution of the cognitive task; and
a processor arrangement configured to analyze the task execution data to determine an alertness value describing an alertness level of the subject during the cognitive test.

The system may further comprise an output interface configured to output a signal based on the determined alertness value.

Some embodiments of the proposed system may further comprise a user interface configured to provide the cognitive test to the subject, the cognitive test prompting execution of the cognitive task.

The system may be remotely located from a user device for cognitive assessment of a subject. In this way, a user (such as a medical professional) may have an appropriately arranged system that can receive task execution data at a location remotely located from the subject. Embodiments may therefore enable a user perform cognitive assessment of a subject using a local system (which may, for example, comprise a portable display device, such as a laptop, tablet computer, mobile phone, PDA, etc.). By way of example, embodiments may provide an application for a mobile computing device, and the application may be executed and/or controlled by a user of the mobile computing device.

The system may further include: a server device comprising the system for cognitive assessment of a subject; and a client device comprising a user-interface. Dedicated data processing means may therefore be employed for the purpose of analyzing task execution data to determine an alertness value describing, thus reducing processing requirements or capabilities of other components or devices of the system.

The system may further include a client device, wherein the client device comprises the input interface processor arrangement and a display unit. In other words, a user (such as a doctor or medical professional) may have an appropriately arranged client device (such as a laptop, tablet computer, mobile phone, PDA, etc.) which processes received task execution data in order to determine an alertness value and generate a display control signal. Purely by way of example, embodiments may therefore provide a monitoring or observation system that enables monitoring of one or more subjects (e.g. patients) from a single location, wherein real-time communication between a subject and monitoring user (e.g. nurse or doctor) is provided and can have its functionality extended or modified according to proposed concepts, for example.

It will be understood that processing capabilities may therefore be distributed throughout the system in different ways according to predetermined constraints and/or availability of processing resources.

Embodiments may be employed in combination with conventional/existing cognitive assessment systems. In this way, embodiments may integrate into legacy systems so as to improve and/or extend their functionality and capabilities. An improved cognitive assessment system may therefore be provided by proposed embodiments.

Thus, there may be proposed concepts for assisting and/or improving cognitive assessment of a subject via the determination/provision of information regarding the alertness of a subject during the performance of a cognitive test. Provision of information about the alertness level of the subject provide additional outcome measures and/or facilitate modification of a cognitive assessment workflow. The proposed concept(s) may thus enable improved (e.g. more accurate) cognitive assessment of a subject.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method 100 for cognitive assessment of a subject according to a proposed embodiment;
Fig. 2 is a graph depicting variations in subject performance over time for three different scenarios;
Fig. 3 is a graph depicting an example of actual measured performance (solid line) of a subject undertaking a cognitive test compared to a reference performance (dashed line);
Fig. 4 depicts an example of a subject's measured performance for different assessment sessions;
Fig. 5 is a simplified block diagram of a system for cognitive assessment of a subject according to an embodiment; and
Fig. 6 is a simplified block diagram of a computer within which one or more parts of an embodiment may be employed.

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figs are merely schematic and are not drawn to scale. It should also be understood that the same reference numerals are used throughout the Figs to indicate the same or similar parts.

The invention proposes concepts for aiding and/or improving cognitive assessment of a subject which may provide the subject and/or a medical professional with greater information surrounding the status of the subject during performance of a cognitive test or assessment. In particular, embodiments of the invention may provide a method and/or system which determines an alertness level of the subject during a cognitive test, and this may be used to support analysis of cognitive assessment results. Further, a determined alertness value may be used to adapt or modify a workflow of neuropsychological test administration, potentially in real time.

In particular, proposed concepts may provide an approach to generating and supplying useful information (regarding subject alertness) for assessment of cognitive performance of a subject. Accordingly, embodiments may be used in relation to treatment selection and/or provide improved Clinical Decision Support (CDS).

For instance, proposed concepts may provide information about subject alertness that may be useful for correcting performance values obtained from cognitive assessments/tests in light of biological cycle data of the subject. For example, a subject may perform better at tasks at certain points in various biological cycles than at other points, as biological cycles impact many internal factors of the subject. Accordingly, insights provided by such data can be used to contextualize performance values of the subject, as well as providing the potential for recommendations regarding the timing of such cognitive examinations.

Overall, it is proposed that determination of an alertness level of a subject during a cognitive test may provide information/insights useful for understanding/analyzing cognitive assessment result. As the alertness of the subject may heavily influence a performance of the subject in executing a cognitive task, knowledge of the subject's level of alertness may be advantageous for accurately understanding performance scores generated by the subject performing a cognitive task, or tasks.

Accurately understanding performance scores relating to cognitive tasks may be particularly useful when diagnosing a subject. Indeed, subjects with neurological conditions may be more heavily impacted by a reduced alertness level. Thus, providing obtaining information about subject alertness, and perhaps adjusting for it, may provide valuable information for properly understanding the state of a subject.

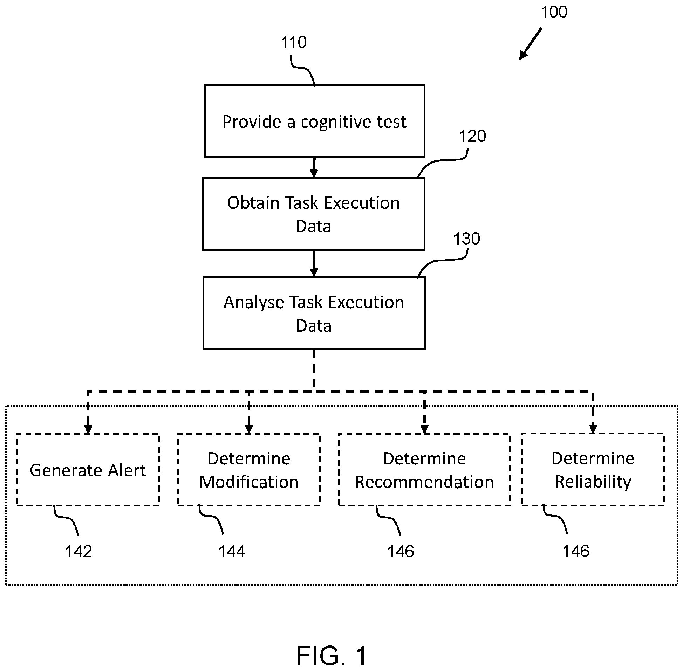

By way of example, a proposed embodiment will now be described with reference to Fig. 1.

Fig. 1 is a simplified flow diagram of a method 100 for cognitive assessment of a subject according to a proposed embodiment. The method may aid and/or assist in the cognitive assessment of subjects (i.e. an individual or patient), such that an improved (i.e. more accurate) assessment of the cognitive abilities of the subject is facilitated. Indeed, such a method may be used alongside existing methods, as will be clear to the skilled person.

The method 100 begins with the step 110 of providing a cognitive test to the subject. The cognitive test is configured to prompt execution of a cognitive task for cognitive assessment. Such a test may, for example, require the subject to answer one or more non-verbal reasoning question, or perform a mental arithmetic exercise. In other examples, a cognitive test may designed to test short term and/or long term memory, reaction speeds and/or comprehension. However, it is to be understood that a wide range of cognitive tests may be employed by proposed methods in order to prompt a subject to execute a cognitive task. The skilled person is well aware of a wide range of existing cognitive tasks suitable for assessing cognitive ability of a subject.

Providing the cognitive task may include simply presenting the cognitive task to the subject, or may comprise a number of steps to induce the subject to perform the cognitive task.

The method then proceeds to the step 120 of obtaining task execution data describing at least one of: a measure of the subject's performance in executing the cognitive task; and a variation of a physiological parameter of the subject during execution of the cognitive task. In this example, the physiological parameter of the subject comprises: eye movement; gaze direction; blinking rate; pupil size; eyelid opening and closing speed; vertical eyelid separation distance; heart rate; blood pressure; respiratory interval; posture; or brain activity.

Next, in step 130, the obtained task execution data is analyzed to determine an alertness value describing an alertness level of the subject during the cognitive test. This may employ one or more of a wide range of known approaches to determining an alertness level of a subject based on variations of a physiological parameter of the subject. Purely by way of example, many prior publications describe techniques and approaches to determining a subject's alertness based on detected variations in their eyes.

Also, for ease of understanding and/or analysis, the determined alertness value may be selected from a group comprising: fatigued/drowsy, bored, engaged, distracted, overloaded/stressed, and impaired. That is, step 130 may further comprise classifying the determined alertness value into one of a set of classifications.

Thus, proposed is an approach for determining a subject's level of alertness during their performance of a cognitive test. This may then be provided leveraged to improve cognitive assessment of the subject. By way of demonstrating the potential uses of the determined level of alertness, Fig. 1 also depicts additional steps (using dashed lines) that may be performed in consideration of the determined level of alertness of the subject.

A first additional step, step 142, comprises generating, based on the determined alertness value, an alert for indicating an alertness level of the subject. The alert may, for example, be generated responsive to the alertness level of the subject meeting a predetermined requirement (e.g. being below a minimum required level). This may, for example, facilitate timely intervention to ensure that a cognitive assessment if performed in an improved or optimal manner.

A second additional step, step 144, comprises determining, based on the determined alertness value, a modification or correction to the subject's execution of at the cognitive task. The modification may be configured to effect dynamic and/or automatic adaptation of an executed task in order to facilitate the provision of more accurate results.

A third additional step, step 146, comprises determining, based on the determined alertness value, cognitive testing recommendation describing provision of a further cognitive test for the subject. Here, the cognitive testing recommendation comprises information describing at least one of: an identifier of the further cognitive test; a timing of provision of the further cognitive test; a modification to a cognitive test to create the further cognitive test a physical task. In this way, workflow adaptation may be facilitated. Such adaptation may comprise: stopping a task; re-doing a task; or performing an alertness enhancing physical exercise.

A fourth additional step, step 146, comprises determining, based on the determined alertness value, a reliability value describing the reliability of the subject's execution of the cognitive task. In this way, supplementary information may be provided for assisting understanding and analysis of assessment results.

Although the embodiment of Fig. 1 is detailed above as employing task execution data which describes a variation of a physiological parameter of the subject during task execution, the task execution data in other embodiments may describe a measure of the subject's performance in executing the cognitive task. The step 130 of analyzing the task execution data may then comprise comparing the measure of the subject's performance with a reference performance value to determine an alertness value. The reference performance value may, for instance, be based on a historic measure of the subject's performance in executing a previous cognitive task. This will be described in further detail below.

It is also noted that the steps of obtaining 120 and analyzing 130 task execution data may be undertaken in real time. That is, proposed embodiments may be implemented so as to provide real-time information regarding the alertness level of the subject.

By way of yet further explanation of the proposed concept(s), a system according to an embodiment may comprise:
(I) A user interface configured to present cognitive tests of a neuropsychological assessment;
(II) An arrangement for determining alertness of the subject from: cognitive test performance data (e.g. the analysis subject performance over time during a test), and/or physiological parameter variation data (e.g. eye-tracking with a camera to provide pupil diameter, eye gaze, and blinking rate, etc,);
(III) a processor configured to analyze and interpret the subject's determined alertness level(s), e.g. number of alertness drops, area under the alertness curve, (total) length of alertness drops, max-min values), and to compare determined alertness values to a predetermined threshold value, longitudinal data or intervention data. The processor may also be configured to suggest changes in the workflow (e.g. postpone a cognitive test to a later time when the subject may be more alert time, execute an alertness-enhancing physical exercise, etc.); and
(IV) An output interface configured to communicate the determined alertness levels, real-time test reliability and/or workflow suggestions to a system, user and/or medical professional.

It is noted that the alertness of the subject can be monitored in several different ways:
(a) A first approach is to calculate alertness drops from the actual recorded performance of the subject, e.g. without requiring additional/supplementary sensing technologies. During administration of cognitive tests, performance of the subject is measured, preferably in real time. The performance can, for example, relate to the response time of the subject, the accuracy of the response, etc. Here, various parameters from a performance curve (e.g. the number of drops from the learning curve, the area-under-the-curve, calculate min-max, etc.) may be calculated and used to determine alertness of the subject.
(b) A second approach is to make use of an additional supplementary sensing technologies, such as eye tracking sensors.

Purely by way of further explanation of the proposed concept(s), various exemplary embodiments that employ different approaches to determining subject alertness will now be described.

### - Calculation of Subject Alertness from a Performance Graph.

In this embodiment, it is proposed to determine an alertness of the subject off-line after all tests from a test battery have been completed by the subject. Alternatively, as a slight refinement in order to be able to adjust workflow, the calculation of subject alertness from the cognitive test performance may be done in (near) real-time or directly after each test from a test battery.

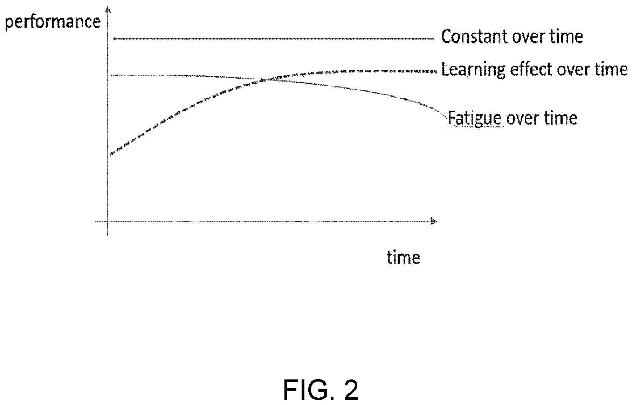

Referring now to Fig. 2, the concept of a 'reference performance', i.e. normal or non-hindered performance will now be described. In particular, Fig. 2 is a graph depicting variations in subject performance over time for three different scenarios.

It is proposed that, if a subject remains alert during a test, their performance during the test will remain roughly constant throughout the test. In contrast, if the alertness of the subject is reduced, it is proposed that this will result in a deviation from the reference performance, and more specifically a reduction in performance. If the subject's alertness is restored, it is to be expected that the test performance will return to the reference performance. As such, negative deviations from the reference performance may be interpreted as a reduction of alertness of the test subject.

In the case that a subject is familiar with a cognitive test (i.e. has carried out the cognitive test several times before), remains alert and does not become fatigued, the reference performance of a test will be relatively close to a constant performance (as depicted by the solid horizontal line in Fig. 2). There may be some random deviations from measurement point to measurement point, but it is proposed that there will generally be no underlying trend in the performance.

Conversely, in the case that a subject is not familiar (or less familiar) with a cognitive test (e.g. carries out the cognitive test for the first time or has carried out the test only occasionally before), remains alert and does not become fatigued, it is proposed that that the reference performance will increase slightly over time (as the subject gains familiarity), as depicted by the dashed curved line in Fig. 2. Again, there be some random deviations from measurement point to measurement point, but the underlying increasing trend in the performance will remain.

In the case that a subject is familiar with a cognitive test (i.e. has carried out the test several times before), remains alert and becomes fatigued during the test, it is proposed that the reference performance will decrease slightly towards the end of the cognitive test (as the subject becomes fatigued), as depicted by the solid curved line in Fig. 2. There may again be some random deviations from measurement point to measurement point, but the underlying decreasing trend in the performance will remain.

It is thus proposed that, in all cases, the reference performance is a roughly constant or at most shows a slight monotonically changing trend.

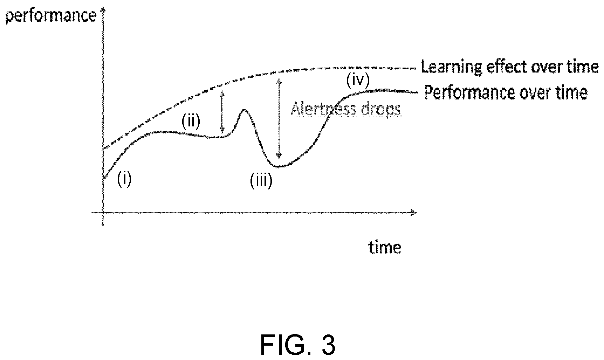

Referring now to Fig. 3, the concept of deviations from the reference performance indicating alertness dips/drops will now be described.

In particular, Fig. 3 is a graph depicting an example of actual measured performance (solid line) of a subject undertaking a cognitive test compared to a reference performance (dashed line). In this example, the subject is not familiar with the test, and as such it will be expected that the performance of the subject may increase during the test (as depicted by the dashed, monotonically increasing line. Thus, in this example, a performance with learning effect is taken as the reference performance.

As can be seen, the performance of the subject does not follow the reference performance:
(i) Initially the performance increases and follows the reference curve as the subject gains familiarity with the test;
(ii) after a short time, however, the actual measured performance drops, indicating that the subject is losing alertness;
(iii) after a short increase in performance, the subject's measured performance drops for a second time, indicating that the subject has lost alertness; and
(iv) towards the end of the test, the subject's performance increases to the highest level and again follows the reference learning curve (e.g. a fairly constant performance at the highest level). This corresponds to the subject becoming fully alert again.

The dips in performance that deviate from the actual learning curve are proposed to be indicative of a loss (i.e. reduction) of alertness of the subject during performance of the cognitive test. As a consequence of this interpretation, it may be possible to more accurately assess the actual performance of the subject in the case that they are fully alert. For example:

In the case where there are considerable portions of the performance which follow/mirror the reference curve (e.g. for Fig. 3, the first 25% and last 25% of the performance graph), the performance during these sections can be taken as the performance during which the subject is fully alert. These portions of the curve may be fitted to a reference curve to even better assess the performance level;

In the case that many short portions of the test are carried out with high performance, a further approach could be to interpolate between the highest performance measurements and use these to fit a reference performance curve; and

In the case that there is no clear following/mirroring of the test performance to any expected reference curve, it may be preferred to propose to repeat the test at a moment that the subject is more alert.

Note that whilst in this case the reference curve is taken from one of a set of reference curves, if the subject is exposed to a series of tests or a repeat of earlier performed tests then these longitudinal measurements may be exploited to better define the reference curve. This will be explained below with reference to another example embodiment.

Dips in performance that deviate from the reference learning curve may also be analyzed to determine various parameter values (e.g. the area under the curve, the min-max calculation, ...). These calculations provide extra outcome measures, that can also be used to adapt a cognitive assessment workflow, for example: stop the test when alertness is below a certain threshold; schedule a demanding test to be performed at an alert moment; or provide an additional physical exercise which increases the alertness level (e.g. cycling on a home trainer before or during administration of a test).

### - Determination of Subject Alertness Based on Data from Additional/Supplementary Sensors

In another embodiment, it is proposed to make use of known physiological parameter sensing technology to provide task execution data from which a subject's alertness level can be determined.

Over longer durations, as is the case in most neuropsychological assessments, the subject will be more likely to exhibit more lapses of alertness over time, and this can also be seen back in various physiological parameter, such as eye movement, pupil diameter, heart rate, blood pressure, respiratory interval, posture, brain activity, etc.

Determining a subject's alertness level from one or more physiological parameters is widely researched and many different approaches are known. For the sake of brevity, such approaches will not be described here in detail. Rather, the skilled person will appreciate that any suitable approach may be used for obtaining and analyzing information about variation in a subject's physiological parameter(s) during execution of a cognitive task in order to determine the subject's alertness level

### - Determination of Alertness from a Combination of Analysis of the Performance Graph and Data from Additional/Supplementary Sensors

Another embodiment may combine the approaches of the preceding two embodiments, namely, to calculate the subject alertness dips from a cognitive test performance curve, and to also calculate the subject's alertness level from variation in one or more physiological parameters of the subject. Combination of both approaches to determining an alertness level of the subject may provide improved (i.e. higher) accuracy. - Longitudinal Comparisons

In some cases, the subject is exposed to many cognitive tests, either in the same test session or carried out at regular intervals in order to assess progression of the subject's performance. If the subject is exposed to a series of tests, or a repeat of earlier performed tests, the longitudinal measurements may be exploited to more accurately define the reference performance.

Hence, in another exemplary embodiment, it is proposed to employ a comparison of longitudinal results between (or even within) sessions of trials:
Compare the results of the present trial with results of earlier trials; and/or
(b) Attempt to establish relative scores between trials at different moments in time by applying a calibration factor

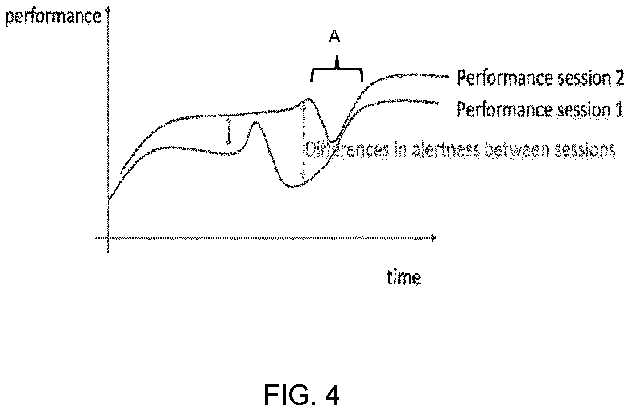

In such a longitudinal situation, it is proposed that the measured performance from previous measurements can be leveraged to define a reference curve for the present performance measurement. This is illustrated in Fig. 4, wherein Fig. 4 depicts a subject's measured performance for different assessment sessions.

In the depicted example of Fig. 4, the performance in the first section ('session 1') is exactly the same as the measure performance depicted in Fig. 3. In this case a reference performance of the subject can be assessed as follows:
(i) Use a proposed approach to assess the performance of the subject as if the subject were fully alert;
(ii) Use the outcome of step (i) above as a reference performance curve for the second ('session 2');
(iii) Assess the fully alert performance of session 2 by comparison with the reference performance curve (i.e. performance from step (i)); and
(iv) Compare the fully alert performance of session 2 to that of session 1.

In this case, it is seen that the performance of session 2 is closer to a monotonically slowly increasing learning curve (apparently the subject is still learning the test). Compared to session 1, the subject now shows only one clear drop in alertness (indicated by "A"). However, the performance of session 2 is increased compared to session 1, as can be seen directly from direct comparison of the first 25% and last 25% of the test, where both tests are indicative of fully alert test subjects. In the case where different parts of the test were to show the fully alert performance, a comparison of fitted reference curves will again allow for a comparison of fully alert subject performance.

### Additional Outcome Measures

In yet another embodiment, the proposed variables are used to provide an additional outcome measure. An analysis of the proposed variables (e.g. pupil diameter, area-under-the-curve alertness dips, ...) can provide insight into the alertness level during execution of a neuropsychological test battery. These insights can help a clinician to interpret the test results, and optionally to adapt the workflow of a test administration. That is, patterns in alertness might hint at specific cognitive disorders.

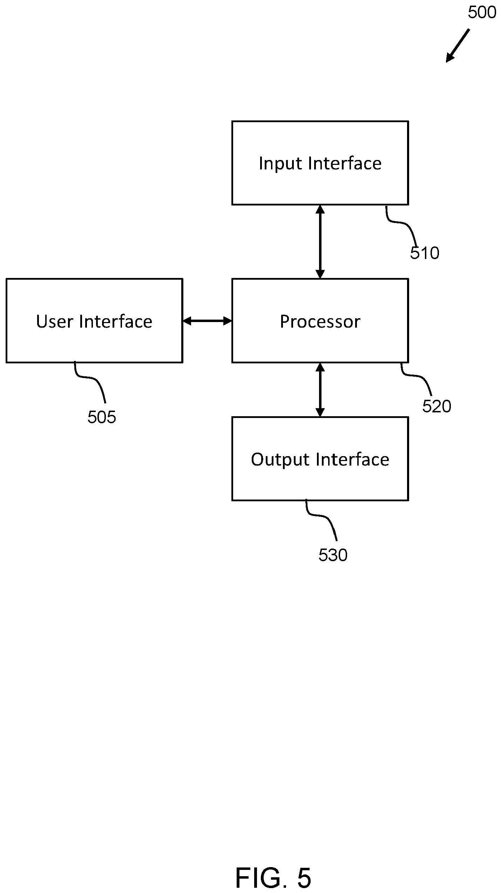

Referring now to Fig. 5, there is depicted a simplified block diagram of a system for cognitive assessment of a subject according to an embodiment. The system 500 comprises a user interface 505 that is configured to provide a cognitive test to the subject, the cognitive test prompting execution of a cognitive task.

The system 500 further comprises an input interface 510 that is configured to obtain task execution data describing at least one of: a measure of the subject's performance in executing the cognitive task for cognitive assessment prompted by the cognitive test; and a variation of a physiological parameter of the subject during execution of the cognitive task. The system 500 also comprises a processor arrangement 520 that is configured to analyze the task execution data to determine an alertness value describing an alertness level of the subject during the cognitive test.

The system 500 yet further comprises an output interface 530 that is configured to output a signal based on the determined alertness value.

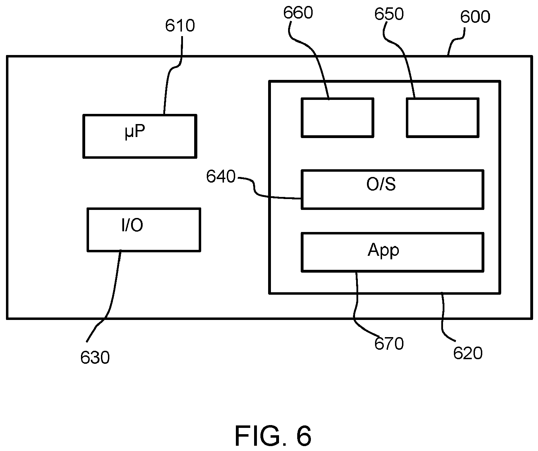

Fig. 6 illustrates an example of a computer 600 within which one or more parts of an embodiment may be employed. Various operations discussed above may utilize the capabilities of the computer 600. For example, one or more parts of a system for providing a subject-specific user interface may be incorporated in any element, module, application, and/or component discussed herein. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620, and one or more I/O devices 670 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor-based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 640, source code 630, and one or more applications 660 in accordance with exemplary embodiments. As illustrated, the application 660 comprises numerous functional components for implementing the features and operations of the exemplary embodiments. The application 660 of the computer 600 may represent various applications, computational units, logic, functional units, processes, operations, virtual entities, and/or modules in accordance with exemplary embodiments, but the application 660 is not meant to be a limitation.

The operating system 650 controls the execution of other computer programs, and provides scheduling, input-output control, file and data management, memory management, and communication control and related services. It is contemplated by the inventors that the application 660 for implementing exemplary embodiments may be applicable on all commercially available operating systems.

Application 660 may be a source program, executable program (object code), script, or any other entity comprising a set of instructions to be performed. When a source program, then the program is usually translated via a compiler (such as the compiler 640), assembler, interpreter, or the like, which may or may not be included within the memory 620, so as to operate properly in connection with the O/S 650. Furthermore, the application 660 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 670 may include input devices such as, for example but not limited to, a mouse, keyboard, scanner, microphone, camera, etc. Furthermore, the I/O devices 670 may also include output devices, for example but not limited to a printer, display, etc. Finally, the I/O devices 670 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 670 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at startup, start the O/S 650, and support the transfer of data among the hardware devices. The BIOS is stored in some type of read-only-memory, such as ROM, PROM, EPROM, EEPROM or the like, so that the BIOS can be executed when the computer 600 is activated.

When the computer 600 is in operation, the processor 610 is configured to execute software stored within the memory 620, to communicate data to and from the memory 620, and to generally control operations of the computer 600 pursuant to the software. The application 660 and the O/S 650 are read, in whole or in part, by the processor 610, perhaps buffered within the processor 610, and then executed.

When the application 660 is implemented in software it should be noted that the application 660 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 660 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-4, and the system of Fig. 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

Data describing an alertness value determined according to an embodiment may be stored on a storage medium. Task execution data according to an embodiment may be stored on the same storage medium or a different storage medium. Such data may be transmitted as a signal modulated onto an electromagnetic carrier wave. The signal may be defined according to a standard for digital communications. The carrier wave may be an optical carrier, a radiofrequency wave, a millimeter wave, or a near field communications wave. It may be wired or wireless.

To the extent that an embodiment is implemented partly or wholly in hardware, the blocks shown in the block diagram of Fig. 5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figs illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figs. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method for cognitive assessment of a subject, the method comprising:
providing (110) a cognitive test to the subject, the cognitive test prompting execution of a cognitive task for cognitive assessment;
obtaining (120) task execution data describing at least one of: a measure of the subject's performance in executing the cognitive task; and a variation of a physiological parameter of the subject during execution of the cognitive task; and
analyzing (130) the task execution data to determine an alertness value describing an alertness level of the subject during the cognitive test.

2. The method of claim 1, further comprising:
based on the determined alertness value, generating (142) an alert for indicating an alertness level of the subject.

3. The method of claim 1 or 2, further comprising:
based on the determined alertness value, determining (144) a modification or correction to the subject's execution of at the cognitive task.

4. The method of any of claims 1 to 3, further comprising:
based on the determined alertness value, determining (146) a cognitive testing recommendation describing provision of a further cognitive test for the subject.

5. The method of claim 4, wherein the cognitive testing recommendation comprises information describing at least one of: an identifier of the further cognitive test; a timing of provision of the further cognitive test; a modification to a cognitive test to create the further cognitive test a physical task.

6. The method of any of claims 1 to 5, further comprising:
based on the determined alertness value, determining (146) a reliability value describing the reliability of the subject's execution of the cognitive task.

7. The method of any of claims 1 to 6, wherein the physiological parameter of the subject comprises: eye movement; gaze direction; blinking rate; pupil size; eyelid opening and closing speed; vertical eyelid separation distance; heart rate; blood pressure; respiratory interval; posture; or brain activity.

8. The method of any of claims 1 to 7, wherein the task execution data describes a measure of the subject's performance in executing the cognitive task,
and wherein analyzing (130) the task execution data comprises: comparing the measure of the subject's performance with a reference performance value to determine an alertness value.

9. The method of claim 8, wherein the reference performance value is based on a historic measure of the subject's performance in executing a previous cognitive task.

10. The method of any of claims 1 to 9, wherein an alertness value is selected from a group comprising: fatigued/drowsy, bored, engaged, distracted, overloaded/stressed, and impaired.

11. The method of any of claims 1 to 10, wherein the steps of obtaining and analyzing task execution data are undertaken in real time.

12. A computer program comprising computer program code means adapted, when said computer program is run on a computer, to implement the method of any of claims 1 to 11.

13. A system for cognitive assessment of a subject, the system comprising:
an input interface (510) configured to obtain task execution data describing at least one of: a measure of the subject's performance in executing a cognitive task for cognitive assessment prompted by a cognitive test; and a variation of a physiological parameter of the subject during execution of the cognitive task; and
a processor arrangement (520) configured to analyze the task execution data to determine an alertness value describing an alertness level of the subject during the cognitive test.

14. The system of claim 13, further comprising:
an output interface (530) configured to output a signal based on the determined alertness value.

15. The system of claim 13 or 14, further comprising:
a user interface (505) configured to provide the cognitive test to the subject, the cognitive test prompting execution of the cognitive task.
